Europäisches Patentamt

European Patent Office

Office européen des brevets

(19)

(11) Numéro de publication : **0 682 948 A1**

## (12) DEMANDE DE BREVET EUROPEEN

(21) Numéro de dépôt : **95400835.5**

(22) Date de dépôt : **12.04.95**

(51) Int. Cl.⁶ : **A61K 33/10, A61K 33/08, A61K 33/42, A61K 35/78**

(30) Priorité : **17.05.94 FR 9405990**

(43) Date de publication de la demande : **22.11.95 Bulletin 95/47**

(84) Etats contractants désignés :
**AT BE CH DE DK ES GB GR IE IT LI LU MC NL PT SE**

(71) Demandeur : **Grimberg, Georges Serge
30 rue Armand Silvestre
F-92400 Courbevoie (FR)**

(72) Inventeur : **Grimberg, Georges Serge
30 rue Armand Silvestre
F-92400 Courbevoie (FR)**

(54) Composition pharmaceutique à base de gomme de guar et d'un antiacide neutralisant, à laquelle peut être ajoutée une série de principes actifs thérapeutiques.

(57)   Composition pharmaceutique à base de gomme de guar et d'un antiacide neutralisant dans laquelle la quantité de gomme de guar doit donner avec de l'eau une suspension suffisamment visqueuse pour se coller à la paroi oeso-gastro-duodénale et se diluer lentement dans les sécrétions oeso-gastro-duodénales, l'association gomme de guar - alcalin aura une quantité de gomme de guar liée à la forme galénique ; une suspention pourra être au point avec 0,2 g de gomme de guar pour 15 ml ou 20 ml d'eau ; alors qu'un comprimé ou une poudre à déliter nécessitera des quantités de gomme de guar plus importantes et même pouvant aller jusqu'à 1g de gomme de guar, la gomme de guar est enrobée de siméthicone, de mono-oléate de sorbitane et de polysorbate 80ᴿ, l'antiacide neutralisant peut être un neutralisant type OMg, $(OH)_2Mg$, OCa, $CO_3Ca$, $A1(OH)_3$, etc, ou même une association de ces antiacides neutralisants, telle que définie par le test de Fortran, à cette composition peut être associé un principe actif thérapeutique complémentaire, physiquement compatible avec cette association.

EP 0 682 948 A1

Jouve, 18, rue Saint-Denis, 75001 PARIS

On connaît déjà la capacité antiacide de la gomme de guar du fait du FR - A - 2 578 423 et de ses demandes de brevet et brevets correspondants dans la plupart des pays du monde.

la gomme guar en tant qu'antiacide peut être associée à d'autres antiacides et notamment des minéraux.

L'objet de la présente invention est la modification très importante d'un antiacide minéral que l'on obtient avec de la gomme de guar.

Un spécialiste des antiacides a été chargé de l'étude de l'activité de l'hydroxyde de magnésium et de l'oxyde de magnésium, en association avec de la gomme de guar enrobée de siméthicone. Il obtient une capacité antiacide remarquable, prolongée, et encore jamais décrite.

L'activité antiacide de trois préparations à base de gomme de guar, enrobée de siméthicone et de sels de magnésium a permis de démontrer l'influence de la gomme sur l'effet antiacide des sels de magnésium. Un témoin sans gomme de guar était fait aussi.

Cette étude a été pratiquée en utilisant le modèle de "l'estomac-duodénum artificiels" en appliquant à ce modèle trois flux de "vidange gastrique simulée".

Conformément à l'invention, dans la composition pharmaceutique à base de gomme de guar et d'un antiacide neutralisant :

a) la quantité de gomme de guar doit donner avec de l'eau une suspension suffisamment visqueuse pour se coller à la paroi oeso-gastro-duodénale et se diluer lentement dans les sécrétions oeso-gastro-duodénales,

b) l'association gomme de guar - alcalin aura une quantité de gomme de guar liée à la forme galénique ; une suspention pourra être au point avec 0,2 g de gomme de guar pour 15 ml ou 20 ml d'eau ; alors qu'un comprimé ou une poudre à déliter nécessitera des quantités de gomme de guar plus importantes et même pouvant aller jusqu'à 1 g de gomme de guar,

c) la gomme de guar est enrobée de siméthicone, de mono-oléate, de sorbitane et de polysorbate 80[R],

d) l'antiacide neutralisant peut être un neutralisant type OMg, $(OH)_2Mg$, OCa, $CO_3Ca$, $Al(OH)_3$, etc, ou même une association de ces antiacides neutralisants, telle que définie par le test de Fortran,

e) on peut associer un principe actif thérapeutique complémentaire, physiquement compatible avec cette association.

Suivant d'autres caractéristiques de l'invention,

a) le principe actif thérapeutique complémentaire pourra être un dérivé fluoré, un sel d'or, de l'acide acétyl-salicylique, un antibiotique, un antiseptique, un anticoagulant.

b) la gomme de guar peut être remplacée par une autre gomme ayant la même capacité de viscosité et d'adhérence à la muqueuse.

Diverses autres caractéristiques de l'invention ressortent de la description détaillée qui suit.

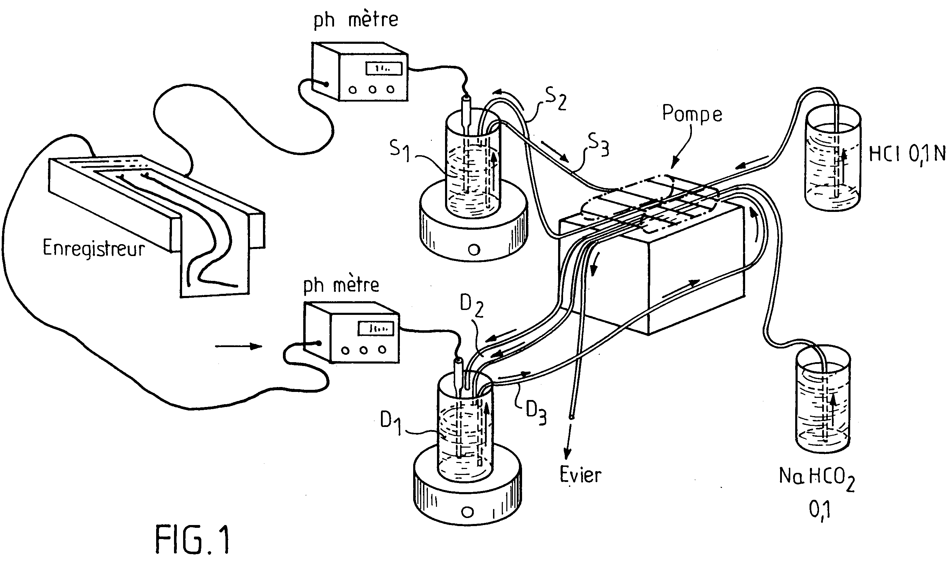

Pour faciliter la compréhension de l'invention, la fig. 1 représente schématiquement un modèle "estomac-duodénum artificiels".

Ces estomac-duodénum artifiels comprennent deux pH-mètres 1 et 2 en liaison d'un côté avec un enregistreur E1 et de l'autre côté avec un récipient S1 représentant le contenu gastrique, un récipient S2 représentant le flux de sécrétion gastrique constant à 3 ml/min (HCl, 0,1 N), S3 représentant le flux de vidange gastrique variable de 1,5, 3,0 ou de 4,5 ml/min, D1 est un récipient représentant le contenu du duodénum, D2 représentant le flux de vidange gastrique entrant dans D1, D3 représentant le flux de vidange de D1 établi pour que D1 reste constant.

Ces flux sont régis par la pompe P.

les pH-mètres et l'enregistreur à deux canaux représentent le système d'enregistrement des pH gastriques et du duodénum D1.

Les études ont été poursuivies sur les produits suuivants qui ont été testés.

Produits testés :

| MGO GUAR 001 | |
|---|---|
| Oxyde de magnésium | 0,5 g |
| Gomme de guar enrobée | 1 g |

| MGO GOM 001 | |
|---|---|
| Hydroxyde de magnésium | 1 g |
| Gomme d guar enrobée | 1 g |

| MGOH GOM 002 | |
|---|---|
| Hydroxyde de magnésium | 0,5 g |
| Gomme de guar enrobée | 1 g |

Les résultats obtenus sont remarquables et présentent pour la première fois la modification d'un neutralisant brutal par un neutralisant tampon qui dure dans le temps.

Les résultats obtenus sont les suivants :

1.MGO GUAR 001

Le tableau I groupe les caractéristiques en site gastrique et duodénal après addition d'un sachet de MGO GUAR 001 dans 100ml d'HCl 0,1 N.

## TABLEAU I

| | | | |
|---|---|---|---|
| Flux de vidange gastrique (ml/min) | 1,5 | 3,0 | 4,5 |
| Site gastrique | | | |
| pH maximum | 3,25 | 1,9 | 4,2 |
| Temps (min) pour retrouver pH 1,0 | 132 | 105 | 75 |
| Millimoles d'acide consommées pour retrouver pH 1,0 | 49,6 | 41,5 | 32,5 |
| Site duodénal | | | |
| Charge acide dans le duodénum (mmol) | 18,3 | 25,72 | 24,9 |
| "Sécrétion alcaline" mmol | 39 | 31,5 | 22,5 |
| Balance $(OH^- - H^+)$ mmol | 20,77 | 5,78 | -2,40 |
| pH duodénal moyen | 6,62 | 6,29 | 6,10 |

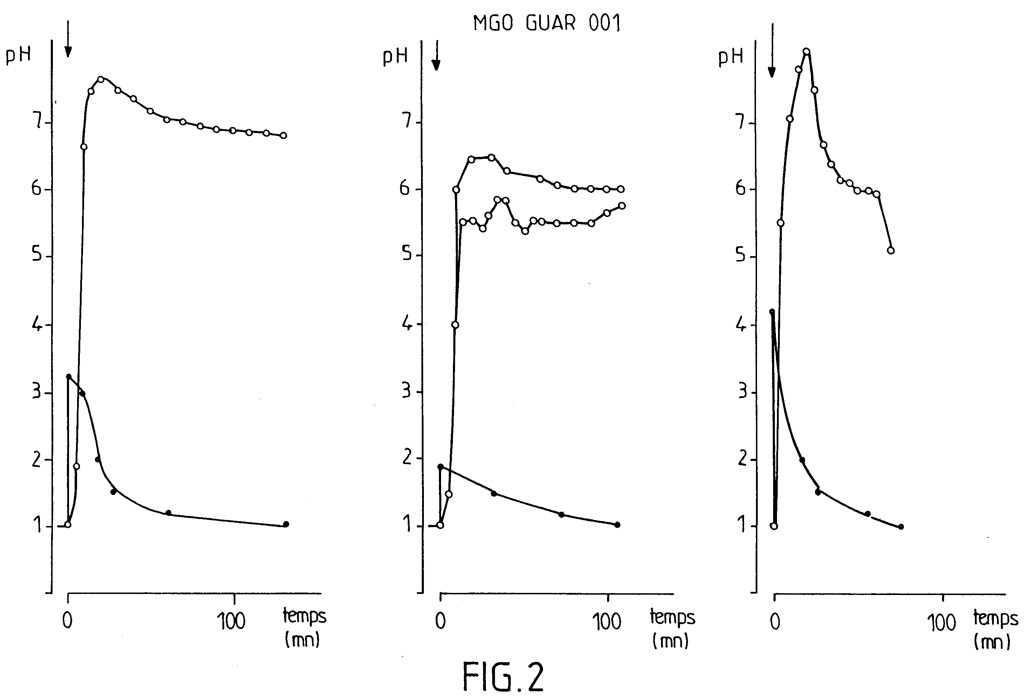

Les tracés de pH-métrie sont reproduits dans la figure 2.

Il existe une relation entre la quantité d'acide consommée pour retrouver pH 1,0 et le rapport entre les flux de sécrétion et de vidange.

$$Y = 11,70\,x + 26,9\ (r = 0,953)$$

où Y représente la quantité d'acide consommée et x le rapport entre les flux de sécrétion et de vidange (3/1,5 = 2,3/3 = 1 et 3/4,5 = 0,66).

Le pH duodénal moyen est corrélé à la balance entre sécrétion alcaline et charge acide.

$Y = 0,022\,x + 6,15\ (r = 1.000)$ où Y représente le pH duodénal et x la balance $OH^- - H^+$. Il apparaît que, pour une valeur nulle de cette balance, le pH duodénal est de 6,15.

## 2.MGO GUAR 001

Les résultats figurent dans le tableau II

TABLEAU II

| Flux de vidange gastrique (ml/min) | 1,5 | 3,0 | 4,5 |
|---|---|---|---|
| Site gastrique | | | |
| pH maximum | 8,7 | 7,1 | 8,9 |
| Temps (min) pour retrouver pH 1,0 | 260 | 225 | 75 |
| Millimoles d'acide consommées pour retrouver pH 1,0 | 88 | 77,5 | 32,5 |
| Site duodénal | | | |
| Charge acide dans le duodénum (mmol) | 28,65 | 53,38 | 18,77 |
| "Sécrétion alcaline" mmol | 78 | 67,5 | 22,5 |
| Balance ($OH^-$ - $H^+$)mmol | 49,35 | 9,12 | 3,73 |
| pH duodénal moyen | 7,13 | 6,70 | 6,61 |

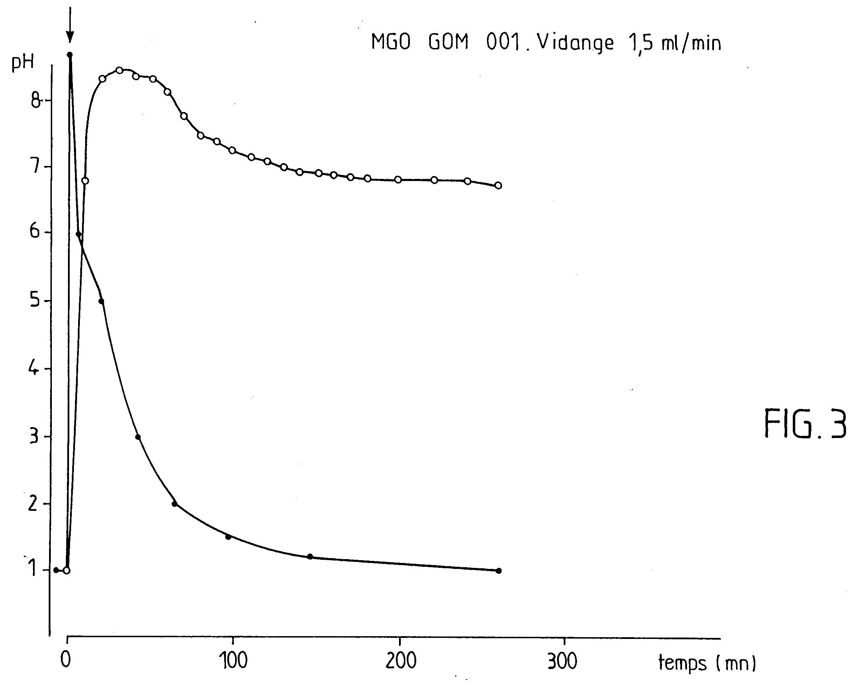

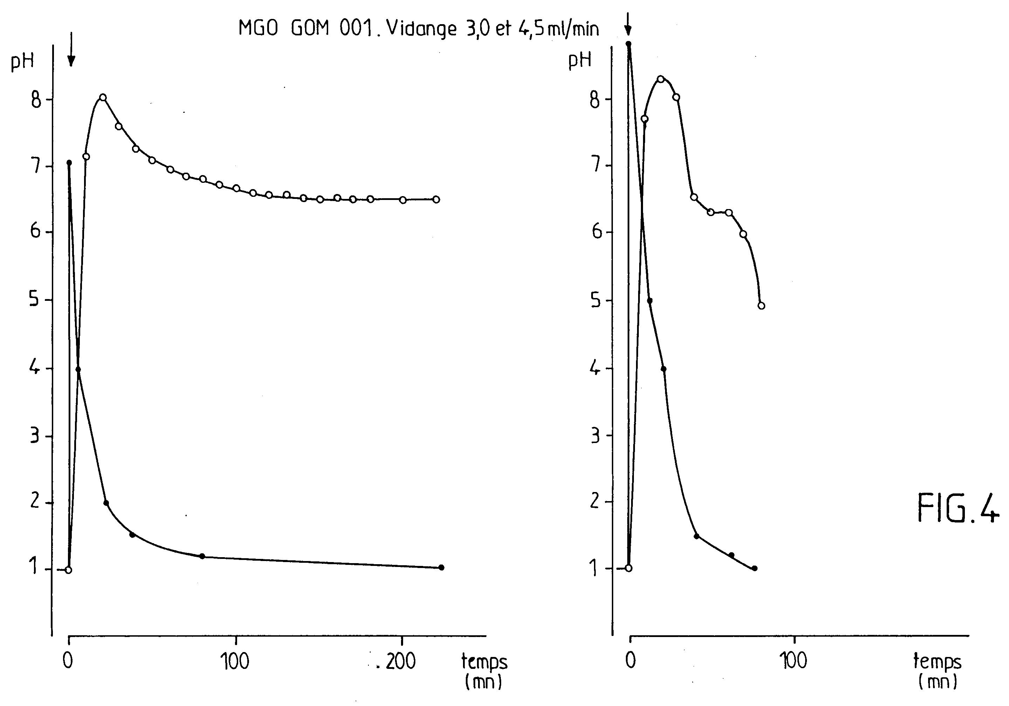

Les tracés de pH-métrie sont reproduits dans les figures 3 et 4.

Relation entre capacité antiacide et rapport des flux de sécrétion et de vidange :

$$Y = 34,4\,x + 24\ (r = 0,812)$$

Relation entre pH duodénal moyen et balance ($OH^-$ - $H^+$) :

$$Y = 0,011\,x + 6,58\ (r = 0,998)$$

Pour une balance de valeur nulle, le pH duodénal moyen est de 6,58.

## 3.MGOH GOM 002

Les résultats figurent dans le tableau III

EP 0 682 948 A1

TABLEAU III

| Flux de vidange gastrique (ml/min) | 1,5 | 3,0 | 4,5 |
|---|---|---|---|
| Site gastrique | | | |
| pH maximum | 2,49 | 3,0 | 1,80 |
| Temps (min) pour retrouver pH 1,0 | 194 | 109 | 60 |
| Millimoles d'acide consommées pour retrouver pH 1,0 | 68,2 | 42,7 | 28 |
| Site duodénal | | | |
| Charge acide dans le duodénum (mmol) | 27,89 | 28,32 | 24,3 |
| "Sécrétion alcaline" mmol | 58,2 | 32,7 | 18 |
| Balance $(OH^- - H^+)$ mmol | 30,31 | 4,38 | -6,30 |
| pH duodénal moyen | 6,22 | 5,73 | 5,68 |

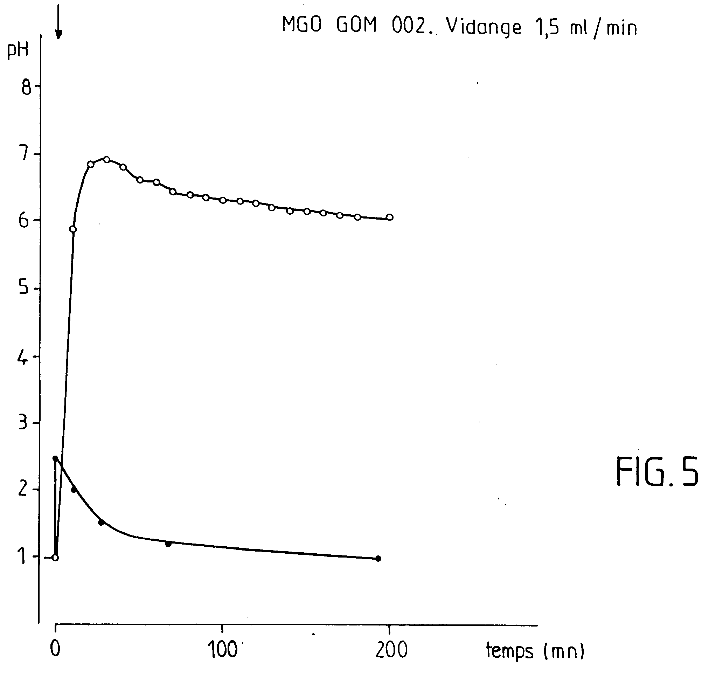

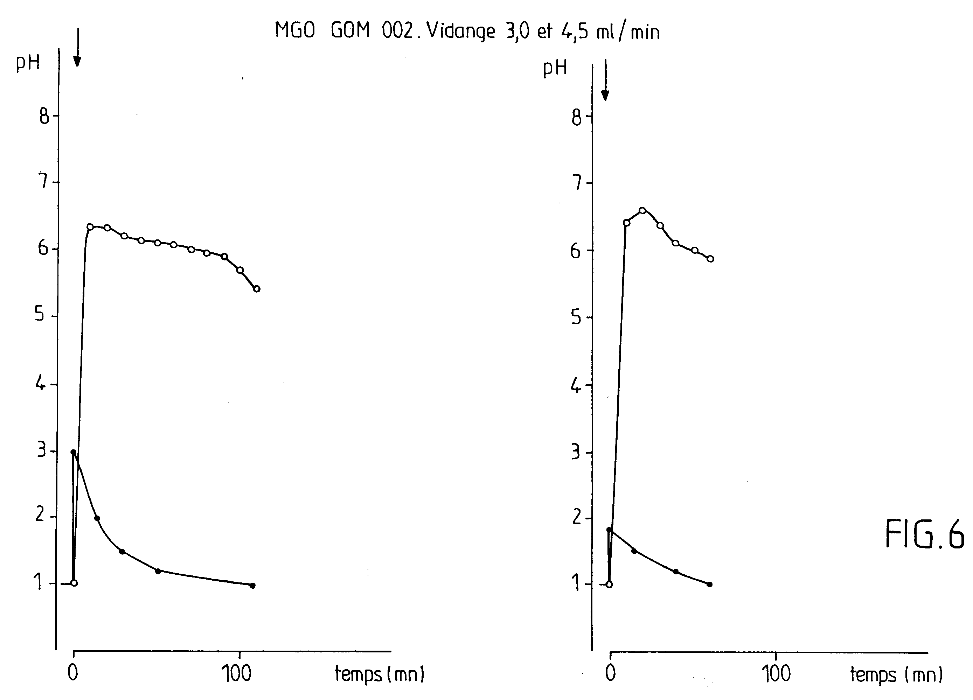

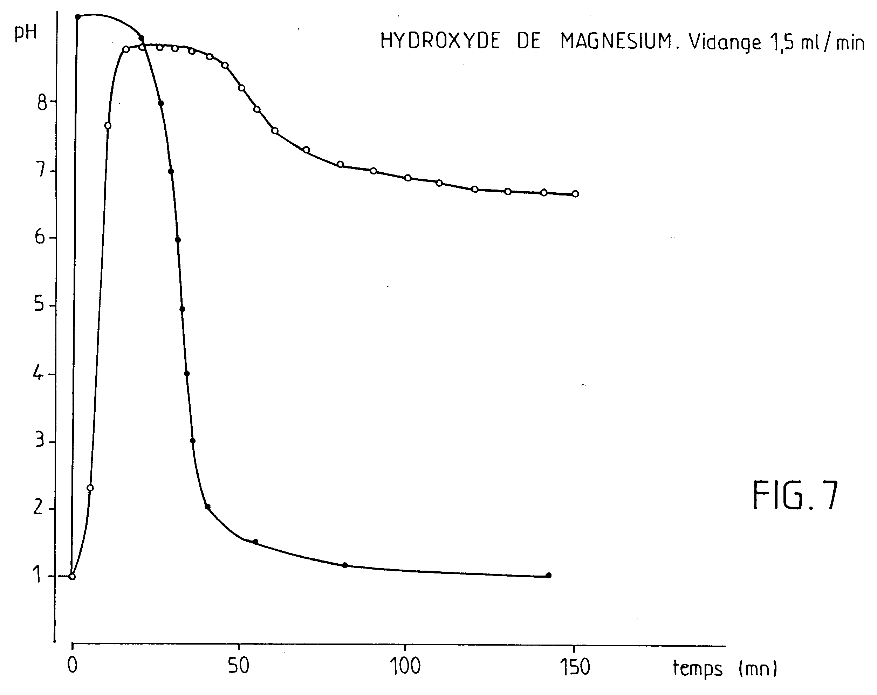

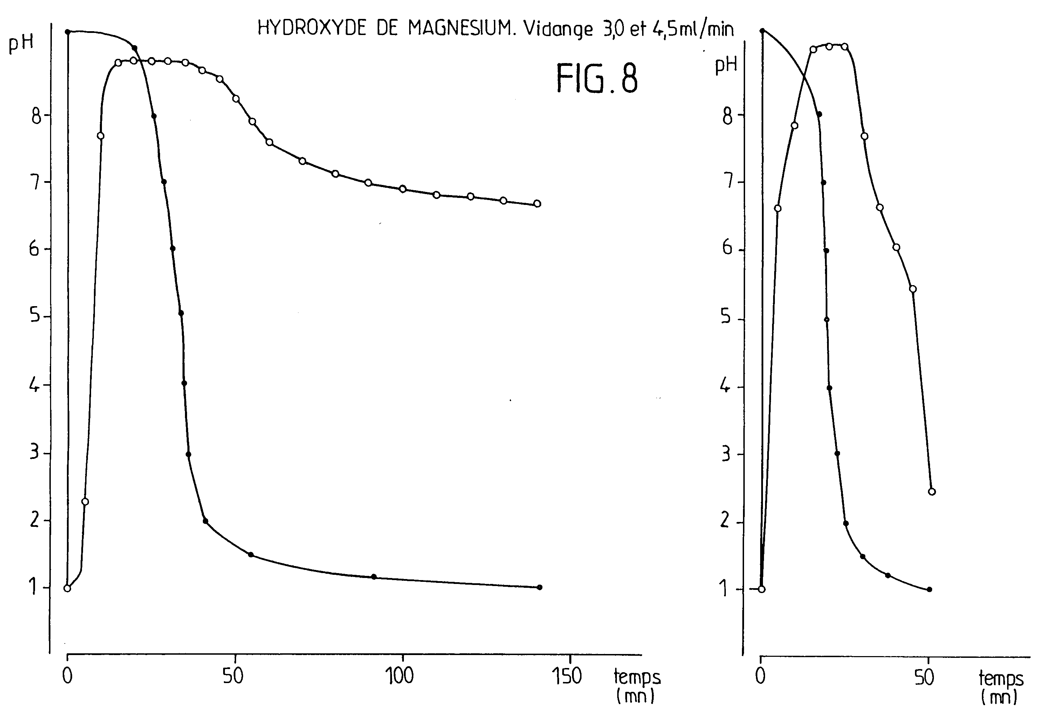

Les tracés de pH-métrie sont reproduits dans les figures 5 et 6.

Relation entre capacité antiacide et rapport des flux de sécrétion et de vidange :

$$Y = 80,7 \, x - 25 \ (r = 0,779)$$

Relation entre pH duodénal moyen et balance $(OH^- - H^+)$ :

$$Y = 0,015 \, x + 5,72 \ (r = 0,979)$$

Pour une balance de valeur nulle, le pH duodénal moyen est de 5,72.

## 4. HYDROXYDE DE MAGNESIUM : 0,8 g

Ce travail a été ajouté pour permettre une comparaison avec les associations de sels de magnésium et de gomme de guar.

Les résultats de ce travail sont groupés dans le tableau IV.

TABLEAU IV.

| 0,8 g de magnésium additionné à 100 ml d'HCl 0,1 N. | | | |
|---|---|---|---|
| Flux de vidange gastrique (ml/min) | 1,5 | 3,0 | 4,5 |
| Site gastrique | | | |
| pH maximum | 9,6 | 9,5 | 9,3 |
| Temps (min) pour retrouver pH 1,0 | 141 | 69 | 50 |
| Millimoles d'acide consommées pour retrouver pH 1,0 | 52,3 | 30,7 | 25 |
| Site duodénal | | | |
| Charge acide dans le duodénum (mmol) | 16,3 | 14,16 | 12,44 |
| "Sécrétion alcaline" mmol | 42,3 | 20,7 | 15 |
| Balance $(OH^- - H^+)$ mmol | 26,17 | 6,54 | 2,51 |
| pH duodénal moyen | 7,19 | 6,98 | 6,52 |

Relation entre quantité d'acide consommée et rapport des flux de sécrétion et de vidange :

$$Y = 20,65 \, x + 10,80 \ (r = 0,948)$$

Relation entre pH duodénal moyen et balance $(OH^- - H^+)$ :

$$Y = 0,022 \, x + 6,63 \ (r = 0,838)$$

L'analyse des résultats démontre à l'évidence l'objet de la présente invention.

La comparaison des équations calculées à partir des corrélations entre la quantité d'acide consommée

5

et les rapports des flux de sécrétion et de vidange dont la pente mesure l'activité antiacide, fait apparaître l'efficacité relative des préparations testées.

| | |
|---|---|
| MGO GUAR 001 | 11,70 |
| MGO GOM 001 | 34,40 |
| MGOH GOM 002 | 80,70 |
| HYDROXYDE DE MAGNESIUM | 20,65 |

soulignant la plus grande activité antiacide de MGOH GOM 002, qui se traduit simultanément par les plus longs délais pour retrouver pH 1,0 par rapport à l'hydroxyde de magnésium et le MGO GUAR 001. Toutefois les délais sont inférieurs à ceux induits par MGO GOM 001 qui tout en manifestant une plus grande quantité d'acide consommée n'aboutit qu'à une puissance antiacide plus faible, cette activité n'étant paradoxalement pas ou peu dépendante des flux intraluminaux gastriques.

L'élévation du pH "intragastrique" est modéré avec MGOH GOM 002, comprise entre 2,5 et 4,5 comme d'ailleurs avec MGO GUAR 001, alors qu'elle est beaucoup plus intense avec l'hydroxyde de magnésium et MGO GOM 001, atteignant respectivement 9,5 et 8,0. Cette élévation brutale du pH du contenu gastrique pourrait in vivo induire la libération de gastrine et un rebond sécrétoire, ce qui est peu vraisemblable avec MGOH GOM 002 et MGO GUAR 001.

Il apparaît donc que la gomme guar associée à une dose d'oxyde ou hydroxyde de magnésium de 0,5 g modifie le comportement des sels de magnésium en freinant leur incidence sur le pH intragastrique et en retardant l'effet de neutralisation, de telle sorte que les délais pour retrouver pH 1,0 soient plus longs correspondant à une activité antiacide plus puissante, comme c'est le cas du meilleur d'entre eux le MGOH GOM 002.

En site duodénal, on peut observer une dépendance étroite du pH moyen du contenu duodénal et de l'équilibre entre la sécrétion alcaline et la charge acide pénétrant dans le duodénum (balance $OH^- - H^+$).

Une valeur élevée du pH duodénal moyen correspond à une économie des bicarbonates sécrétés par un effet antiacide neutralisant en site gastrique. Cette valeur du pH duodénal peut être évaluée par la valeur de pH lorsque la balance ($OH^- - H^+$) est de valeur nulle.

Ces valeurs sont respectivement, pour :

| | |
|---|---|
| MGO GUAR 001 | 6,15 |
| MGO GOM 001 | 6,58 |
| MGOH GOM 002 | 5,72 |
| HYDROXYDE DE MAGNESIUM | 6,63 |

L'économie des bicarbonates est plus intense avec l'hydroxyde de magnésium et décroit avec MGO GOM 001, MGO GUAR 001 et MGOH GOM 002 dont la valeur de 5,72 peut correspondre à une valeur physiologique du pH duodénal in vivo.

En conclusion, les trois préparations présentent tant en site gastrique où elles développent une activité antiacide puissante qu'en site duodénal où elles induisent un pH duodénal moyen compatible avec les valeurs physiologiques, les critères de bons antiacides.

L'association de gomme de guar avec des oxydes et hydroxydes de magnésium aboutit à des préparations antiacides respectueuses du pH intragastrique, puissantes dans la consommation d'acide et ne modifiant pas les conditions physiologiques du pH duodénal.

Dans une autre étude, la capacité antiacide d'une suspension d'antiacide plus de la gomme de guar enrobée de siméthicone donne une durée d'action de plus de 5 heures.

La présente invention permet évidemment de remplacer un alcalin tel que l'aluminium qui est toxique par un alcalin bénéfique tel que le magnésium et même le calcium, par exemple du carbonate de calcium ; il est à noter que le siméthicone va, dans le cadre du $CO_2$ formé, jouer un rôle important comme antimousse.

Dans la présente invention, la gomme guar enrobée de siméthicone transforme un neutralisant minéral en un antiacide à action qualifiée de "physiologique".

Dans la présente invention, l'acide chlorhydrique stomacale va transformer l'alcalin magnésium en chlorure de magnésium soluble, donc ce produit qui a une capacité antiacide remarquablement physiologique va aussi servir de magnésothérapie.

De même, le dérivé calcique va donner un chlorure de calcium soluble et servir de calcithérapie.

Un antiacide de capacité physiologique non toxique et de longue durée d'action va délivrer lentement dans le temps le cation que l'on veut donner à l'organisme, ce qui va permettre une meilleure absorption et une meilleure pénétration cellulaire.

Cette gomme de guar enrobée de siméthicone associée à un alcalin est un antiacide physiologique et un minéralisant à action prolongée et peut être associée à un autre principe actif.

Par exemple, au carbonate de calcium peuvent être ajoutés 100 mg de monofluorophosphate.

On peut également prendre l'exemple de l'acide acétylsalicylique :
- Gomme de guar enrobée          1 g
- $(Ho)_2$ Mg          0,5 g
- Acide acétylsalicylique          0,5 g

On obtient un antiacide à capacité physiologique qui détoxique l'aspirine et permet une lente diffusion de son activité thérapeutique.

Cette association gomme de guar alcalin peut être faite avec un très grand nombre de principes actifs : antibiotiques, sels d'or, etc.

Cette association avec un alcalin peut être faite avec d'autres gommes qui permettent d'obtenir une viscosité convenable et une adhérence suffisante sur la muqueuse.

## Revendications

**1 -** Composition pharmaceutique à base de gomme de guar et d'un antiacide neutralisant dans laquelle :
a) la quantité de gomme de guar doit donner avec de l'eau une suspension suffisamment visqueuse pour se coller à la paroi oeso-gastro-duodénale et se diluer lentement dans les sécrétions oeso-gastro-duodénales,
b) l'association gomme de guar - alcalin aura une quantité de gomme de guar liée à la forme galénique ; une suspention pourra être au point avec 0,2 g de gomme de guar pour 15 ml ou 20 ml d'eau ; alors qu'un comprimé ou une poudre à déliter nécessitera des quantités de gomme de guar plus importantes et même pouvant aller jusqu'à 1g de gomme de guar,
c) la gomme de guar est enrobée de siméthicone, de mono-oléate de sorbitane et de polysorbate 80[R],
d) l'antiacide neutralisant peut être un neutralisant type OMg, $(OH)_2Mg$, OCa, $CO_3Ca$, $Al(OH)_3$, etc, ou même une association de ces antiacides neutralisants, telle que définie par le test de Fortran,
e) on peut associer un principe actif thérapeutique complémentaire, physiquement compatible avec cette association.

**2 -** Composition selon la revendication 1, caractérisée en ce que le principe actif thérapeutique complémentaire pourra être un dérivé fluoré, un sel d'or, de l'acide acétyl-salicylique, un antibiotique, un antiseptique, un anticoagulant, etc.

**3 -** Composition selon la revendication 1, caractérisée en ce que la gomme de guar peut être remplacée par une autre gomme ayant la même capacité de viscosité et d'adhérence à la muqueuse.

ph mètre

ph mètre

Enregistreur

$S_1$  $S_2$  $S_3$

Pompe

HCl 0,1 N

$D_1$  $D_2$  $D_3$

Evier

$NaHCO_2$ 0,1

FIG.1

EP 0 682 948 A1

MGO GUAR 001

FIG.2

MGO GOM 001. Vidange 1,5 ml/min

FIG.3

EP 0 682 948 A1

MGO GOM 001. Vidange 3,0 et 4,5 ml/min

FIG.4

EP 0 682 948 A1

MGO GOM 002. Vidange 1,5 ml / min

FIG. 5

EP 0 682 948 A1

MGO  GOM 002. Vidange 3,0 et 4,5 ml/min

FIG.6

EP 0 682 948 A1

HYDROXYDE DE MAGNESIUM. Vidange 1,5 ml / min

FIG. 7

HYDROXYDE DE MAGNESIUM. Vidange 3,0 et 4,5ml/min

FIG.8

EP 0 682 948 A1

**Office européen**

**des brevets**

## RAPPORT PARTIEL
## DE RECHERCHE EUROPEENNE

qui selon la règle 45 de la Convention sur le brevet
européen est considéré, aux fins de la procédure ultérieure
comme le rapport de la recherche européenne

Numero de la demande

EP 95 40 0835

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,D | FR-A-2 578 423 (GRIMBERG, GEORGES SERGE) * le document en entier * --- | 1-3 | A61K33/10 A61K33/08 A61K33/42 A61K35/78 |
| X,P | EP-A-0 601 908 (GRIMBERG, GEORGES SERGE) * page 3; exemple 3 * --- | 1-3 | |
| A | US-A-3 382 150 (GRASS, GEORGE M.) * revendications * ----- | 1-3 | |

**DOMAINES TECHNIQUES RECHERCHES (Int.Cl.6)**

A61K

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes:
Revendications ayant fait l'objet de recherches incomplètes:
Revendications n'ayant pas fait l'objet de recherches:
Raison pour la limitation de la recherche:

voir feuille supplémentaire C

| Lieu de la recherche | Date d'achèvement de la recherche | Examinateur |
|---|---|---|
| LA HAYE | 24 Août 1995 | Leherte, C |

16

EP 95 40 0835

-C-

RECHERCHE INCOMPLETE

Revendication ayant fait l'objet de recherches complètes: 1

Revendications ayant fait l'objet de recherches incomplètes: 2,3

En vue du très grand nombre de composés revendiqués, la recherche a été faite selon l'idée générale de la demande et a surtout été concentré sur les composés mentionnés spécifiquement dans les exemples.